Europäisches Patentamt
European Patent Office
Office européen des brevets

(11) Veröffentlichungsnummer: **0 000 380 A1**

## (12) EUROPÄISCHE PATENTANMELDUNG

(21) Anmeldenummer: **78100322.3**

(22) Anmeldetag: **07.07.78**

(51) Int. Cl.²: **C07D499/02**, A61K31/43, A23K1/17

(30) Priorität: **15.07.77 DE 2732104**

(43) Veröffentlichungstag der Anmeldung: **24.01.79 Patentblatt 79/2**

(84) Benannte Vertragsstaaten: **BE CH DE FR GB NL SE**

(71) Anmelder: **Bayer Aktiengesellschaft, Zentralbereich Patente,Marken und Lizenzen Bayerwerk, D-5090 Leverkusen 1 (DE)**

(72) Erfinder: **Feyen, Peter, Dr., Mozartstrasse 1, D-4020 Mettmann (DE)**
**König, Hans-Bodo, Dr., Herberts Katernberg 10, D-5600 Wuppertal 1 (DE)**
**Metzger, Karl Georg, Dr., Pahlkestrasse 1, D-5600 Wuppertal 1 (DE)**

# (54) Penicilline, Verfahren zu ihrer Herstellung und ihre Verwendung.

(57) Penicilline der Formel

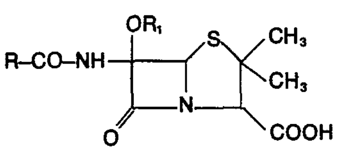

I

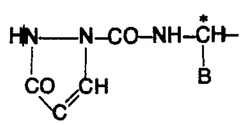

Verfahren zu ihrer Herstellung und ihre Verwendung als antibakterielle Mittel und als Mittel zur Förderung des Wachstums und zur Verbesserung der Futterverwertung bei Tieren.

In der Formel I, ist $R_1$ Wasserstoff oder gegebenenfalls substituiertes Alkyl, Aralkyl, Aryl, Cycloalkyl, Heterocyclylalkyl oder Heterocyclyl,

R ist

R ist

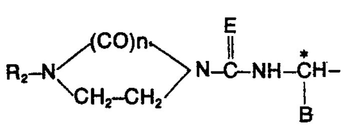 oder

EP 0 000 380 A1

BAYER AKTIENGESELLSCHAFT
Zentralbereich
Patente, Marken und Lizenzen

5090 Leverkusen, Bayerwerk
PG/by

# Penicilline, Verfahren zu ihrer Herstellung und ihre Verwendung

Die vorliegende Erfindung betrifft Penicilline, Verfahren zu ihrer Herstellung sowie ihre Verwendung als Arzneimittel, insbesondere als antibakterielle Mittel und als Mittel zur Förderung des Wachstums und zur Verbesserung der Futterverwertung bei Tieren.

Die erfindungsgemäßen Penicilline haben eine breite, antibakterielle Wirksamkeit. Sie sind wirksam gegen Gram-negative und Gram-positive Keime. Ferner zeigen die erfindungsgemäßen Verbindungen eine große Stabilität gegen ß-Lactamase bzw. eine hohe Wirksamheit gegen solche Bakterien, die ß-Lactamase bilden. Die erfindungsgemäßen Penicilline haben fernerhin die Eigenschaft, auch andere Penicilline vor der Zerstörung durch ß-Lactamase zu schützen.

Die erfindungsgemäßen Verbindungen entsprechen der allgemeinen Formel (I)

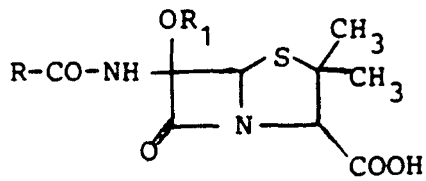

(I)

in der

$R_1$ Wasserstoff, gegebenenfalls substituiertes geradkettiges oder verzweigtes Alkyl mit vorzugsweise 1 bis 6, insbesondere 1 bis 3 Kohlenstoffatomen, gegebenenfalls substituiertes geradkettiges oder verzweigtes Alkenyl mit vorzugsweise 2 bis 6, insbesondere 3 oder 4 Kohlenstoffatomen, geradkettiges oder verzweigtes Alkinyl mit der Dreifachbindung in der 2-Stellung und mit vorzugsweise 3 bis 6 Kohlenstoffatomen, gegebenenfalls substituiertes Cycloalkyl mit vorzugsweise 3 bis 9, insbesondere 3 bis 6, ganz besonders bevorzugt 3 oder 4 Kohlenstoffatomen, gegebenenfalls substituiertes Aralkyl mit vorzugsweise 7 bis 10 Kohlenstoffatomen, gegebenenfalls substituiertes Aryl mit vorzugsweise 6 bis 9 Kohlenstoffatomen, einen gegebenenfalls substituierten Heterocyclylalkyl oder heterocyclischen Rest, der in beiden Fällen 3-, 4-, 5- oder 6-gliedrig sein kann, der Sauerstoff, Schwefel und Stickstoff als Heteroatome einzeln oder kombiniert oder mehrfach einzeln enthalten kann und gesättigt oder ungesättigt sein kann, und

R einen Rest der allgemeinen Formeln (II) oder (IIa)

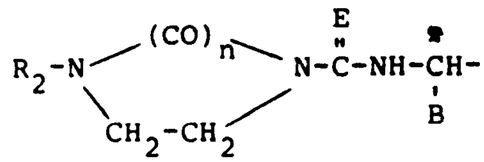

(II)

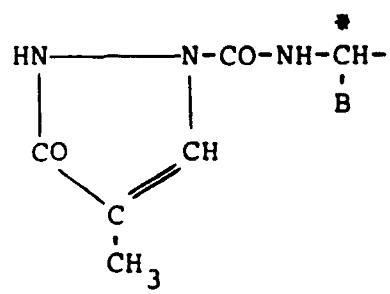

(IIa)

darstellt, in denen C * ein Chiralitätszentrum ist, das in der R- oder S-Form vorliegen kann und E Sauerstoff oder Schwefel, vorzugsweise Sauerstoff bedeutet,

B Phenyl, substituiertes Phenyl, insbesondere Hydroxyphenyl, vorzugsweise p-Hydroxyphenyl, Cyanphenyl, insbesondere p-Cyanphenyl, Methylsulfonylphenyl, insbesondere p-Methylsulfonylphenyl, Methylsulfonylaminophenyl, Methoxyphenyl, Halogenphenyl, insbesondere Fluorphenyl oder Chlorphenyl, 1-Cyclohexenyl-(1) und 1,4-Cyclohexadienyl-(1) darstellt,

n 1 oder 2 bedeutet und

$R_2$ für den Fall, daß n = 1 und $R_1$ = Methyl ist, Wasserstoff, gegebenenfalls substituiertes geradkettiges oder verzweigtes Alkyl mit vorzugsweise 1 bis 4, insbesondere 1 oder 2 Kohlenstoffatomen, gegebenenfalls

substituiertes geradkettiges oder verzweigtes Alkenyl mit der Doppelbindung in der 2-, 3-, 4- oder 5-Stellung mit vorzugsweise 3 bis 6, insbesondere 3 oder 4 Kohlenstoffatomen, geradkettiges oder verzweigtes Alkinyl mit der Dreifachbindung in der 2-, 3- oder 4-Stellung, einem Rest der allgemeinen Formel (III) oder (IV)

$R_3$-CO- , $R_4$-$SO_2$-

(III) (IV)

worin $R_3$ und $R_4$ gleich $R_2$ sind oder

$R_3$ Wasserstoff, Niederalkoxy mit vorzugsweise 1 bis 4, insbesondere 1 oder 2 Kohlenstoffatomen, $R_3$ und $R_4$ Amino, Niederalkylamino mit vorzugsweise 1 bis 4, insbesondere 1 oder 2 Kohlenstoffatomen, Diniederalkylamino mit vorzugsweise 2 bis 6, insbesondere 2 bis 4 Kohlenstoffatomen,bedeutet und wo beide Alkylreste miteinander direkt oder über ein Heteroatom wie O, N oder S unter Bildung eines heterocyclischen Ringes mit vorzugsweise 4 bis 6, insbesondere 5 oder 6 Ringgliedern verbunden sein können, worin ferner $R_3$ und

$R_4$ gegebenenfalls substituiertes Cycloalkyl mit vorzugsweise 3 bis 9, insbesondere 3 bis 6 ganz besonders bevorzugt 3 oder 4 Kohlenstoffatomen, gegebenenfalls substituiertes Aralkyl mit vorzugsweise 7 bis 9

Kohlenstoffatomen, gegebenenfalls substituiertes Aryl mit vorzugsweise 6 bis 8 Kohlenstoffatomen, einen gegebenenfalls substituierten heterocyclischen Rest bedeuten, der 3-, 4-, 5- oder 6-gliedrig sein kann, der Sauerstoff, Schwefel und Stickstoff als Heteroatom einzeln kombiniert oder mehrfach enthalten kann und gesättigt oder ungesättigt sein kann und insbesondere Furan, Thiophen, Pyrrol, Oxazol, Isoxazol, Thiazol, Isothiazol, Pyrazol, Imidazol, Pyridin und Pyrimidin darstellt.

$R_2$ für den Fall, das n = 2 oder $R_1$ von Methyl verschieden ist

Wasserstoff, gegebenenfalls substituiertes geradkettiges oder verzweigtes Alkyl mit vorzugsweise 1 bis 6, insbesondere 1 bis 3 Kohlenstoffatomen, gegebenenfalls substituiertes geradkettiges oder verzweigtes Alkenyl mit vorzugsweise 2 bis 6, insbesondere 3 oder 4 Kohlenstoffatomen, geradkettiges oder verzweigtes Alkinyl mit der Dreifachbehandlung in der 2-Stellung und mit vorzugsweise 3 bis 6 Kohlenstoffatomen, gegebenenfalls substituiertes Cycloalkyl mit vorzugsweise 3 bis 9, insbesondere 3 bis 6, ganz besonders bevorzugt 2 oder 4 Kohlenstoffatomen, gegebenenfalls substituiertes Aralkyl mit vorzugsweise 7 bis 10 Kohlenstoffatomen, gegebenenfalls substituiertes Aryl mit vorzugsweise 6 bis 9 Kohlenstoffatomen, einen gegebenenfalls substituierten Heterocyclylalkyl oder heterocyclischen Rest, der in beiden Fällen 3-, 4-, 5- oder 6-gliedrig sein kann, der Sauerstoff, Schwefel und

Stickstoff als Heteroatome einzeln oder kombiniert oder mehrfach einzeln enthalten kann und gesättigt oder ungesättigt sein kann, sowie die Salze der Verbindungen gemäß Formel (I), insbesondere die pharmazeutisch verwendbaren Salze.

Pharmazeutisch verwendbare Salze der Verbindungen der Formel (I) sind Salze dieser Verbindungen mit anorganischen und organischen Basen an der sauren Carboxylgruppe bzw. den sauren Carboxyl- und Sulfonsäuregruppen. Als Basen können hierzu alle in der pharmazeutischen Chemie, insbesondere in der Chemie der Antibiotika, üblicherweise verwendeten Basen eingesetzt werden. Als anorganische Basen seien beispielhaft genannt: Alkali- und Erdalkalihydroxide, Alkali- und Erdalkalicarbonate und Alkalihydrogencarbonate, wie Natrium- und Kaliumhydroxid, Calcium- und Magnesiumhydroxid, Natrium- und Kaliumcarbonat, Calciumcarbonat, Natrium- und Kaliumhydrogencarbonat; Aluminiumhydroxid und Ammoniumhydroxid. Als organische Amine können primäre, sekundäre und tertiäre aliphatische Amine sowie heterocyclische Amine eingesetzt werden. Beispielhaft seien genannt: Di- und Triniedrigalkylamine, z.B. Diäthylamin, Triäthylamin, Tri-ß-hydroxyäthylamin, Procain, Dibenzylamin, N,N'-Dibenzyläthylendiamin, N-Benzyl-ß-phenyl-äthylamin, N-Methyl- und N-Äthylmorpholin, 1-Ephenamin, Dehydroabietylamin, N,N'-Bis-dehydroabietyläthylendiamin, N-Niedrigalkylpiperidin. Auch sogenannte basische Aminosäuren wie Lysin oder Arginin können vorteilhaft als Basen Verwendung finden. Besonders bevorzugte Salze sind die Natriumsalze.

Mögliche Substituenten für $R_1$, $R_2$, $R_3$ und $R_4$ als Alkyl-, Alkenyl-, Cycloalkyl-, Aryl-, Aralkyl-, Heterocyclyl- und gegebenenfalls Heterocyclylalkyl-Rest sind Halogen, vorzugsweise Fluor, Chlor und Brom, vorzugsweise Fluor und Chlor, Niederalkoxy mit vorzugsweise 1 oder 2 Kohlenstoffatomen, geschützte Hydroxy-, Amino-, Niederalkylamino-Gruppen mit vorzugsweise 1 oder 2 Kohlenstoffatomen und Carbonyl- und Sulfonsäuregruppen, Diniederalkylaminogruppen mit vorzugsweise 2 bis 4 Kohlenstoffatomen, Ketogruppen und Estergruppen mit vorzugsweise 2 oder 3 Kohlenstoffatomen, Carbonyl- und Sulfamylreste, heterocyclische Reste wie Furanyl, Thienyl, Pyrrolyl und Pyridinyl. Mögliche Substituenten $R_1$, $R_2$, $R_3$ und $R_4$ als heterocyclischer Rest bzw. Heterocyclylalkyl-Rest sind Alkyl mit vorzugsweise 1 oder 2 Kohlenstoffatomen oder durch eine Carboxyl- oder Sulfonsäuregruppe substituierte Methyl- oder Äthylgruppen.

Es wurde weiterhin gefunden, daß man die Verbindungen der Formel (I) erhält, wenn man Penicilline der Formel (V)

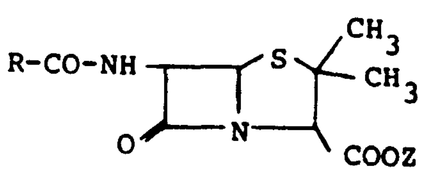

(V)

in der

R die oben angegebene Bedeutung besitzt und

Z Wasserstoff, Acetoxymethyl, eine leicht entfernbare esterbildende Gruppe oder den kationischen Rest einer Base, vorzugsweise eines Alkali- oder Erdalkalimetallhydroxids, bezeichnet, mit 2-10 Äquivalenten pro Äquivalent des Penicillins einer Base in Anwesenheit eines Überschusses eines Alkohols der Formel $R_1OH$, in der $R_1$ die oben angegebene Bedeutung besitzt, in einem inerten organischen Lösungsmittel umsetzt, zwischen 1 bis 8 Äquivalente eines N-Halogenierungsmittels zufügt und die Verbindung der Formel (I), gegebenenfalls nach vorheriger Abspaltung der Säureschutzgruppe und/oder Überführung in ein Salz isoliert. Insbesondere die direkte Arbeitsweise, d.h. die Umsetzung von ungeschützten Verbindungen der Formel (V) bietet Vorteile bezüglich der Einfachheit der Reaktionsführung.

Die Ausgangsprodukte der Formel (V) sowie der Formel $R_1$-OH sind bekannt oder können nach bekannten Verfahren hergestellt werden.

An Hand des folgenden Reaktionsschemas sei das Verfahren beispielhaft erläutert:

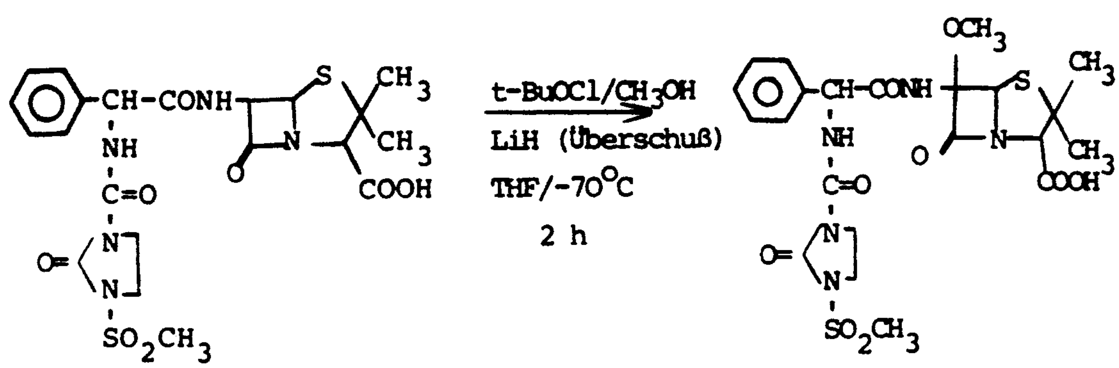

Bei dem erfindungsgemäßen Verfahren werden als N-Halogenierungsmittel, vorzugsweise positives Chlor übertragende Verbindungen, wie t-Butylhypochlorit oder Chloracetamid verwendet.

Als Basen eignen sich komplexe, und einfache, vorzugsweise jedoch einfache Alkali- und Erdalkalihydride, metallorganische Verbindungen, sowie Grignard-Verbindungen. Beispielsweise seien genannt: Lithiumhydrid, Natriumhydrid, Butyllithium, Phenyl-Lithium, Alkyl-Magnesiumbromide, beispielsweise Methyl-Magnesiumbromid, oder andere bekannte Säurebindemittel wie Alkali- und Erdalkalialkoholate oder -carbonate, Alkali- und Erdalkali-bicarbonate oder -oxide wie beispielsweise Natriumbicarbonat oder andere Säurebindemittel wie Borax oder offenkettige oder cyclische organische Basen wie Trialkyl- oder Aralkylamine oder cyclische Amidine wie 2,3,4,6,7,8-hexahydro-pyrrolo[1,2-a]pyrimidin (DBN) oder 2,3,4,6,7,8.9.10-Octahydro-pyrimido[1,2-a]azepim (DBU).

Als Lösungsmittel eignen sich beispielsweise offenkettige oder cyclische Äther, aliphatische und aromatische Kohlenwasserstoffe oder Halogenkohlenwasserstoffe oder die Alkohole $R_1OH$. Besonders geeignet ist Tetrahydrofuran.

Die Reaktionstemperaturen sind möglichst unter $0^oC$ zu halten, vorzugsweise zwischen $-100^oC$ und $-45^oC$.

Bei der Durchführung des erfindungsgemäßen Verfahrens wird 1 Moläquivalent der Verbindung der allgemeinen Formel V zu dem Gemisch aus vorzugsweise 3 bis 8 Moläquivalenten Base,

2 bis über (falls es selbst als Lösungsmittel dient) 100 Moläquivalenten des Alkohols $R_1OH$ und dem Lösungsmittel bei den angegebenen tiefen Temperaturen gegeben und anschließend sofort 1 bis 5, vorzugsweise 1 bis 2 Moläquivalent Halogenierungsmittel (z.B. t-Butylhypochlorit) hinzugefügt. Die Reaktionszeit beträgt im allgemeinen etwa 10 Minuten bis mehrere Stunden bei -70°C.

Zunächst werden dann bei neutralem pH Verunreinigungen durch Extraktion mit einem organischen Lösungsmittel entfernt. Dann wird das erfindungsgemäße Verfahrensprodukt bei saurem pH aus dem Reaktionsgemisch extrahiert.

Als Beispiele für erfindungsgemäße Verbindungen seien aufgeführt:

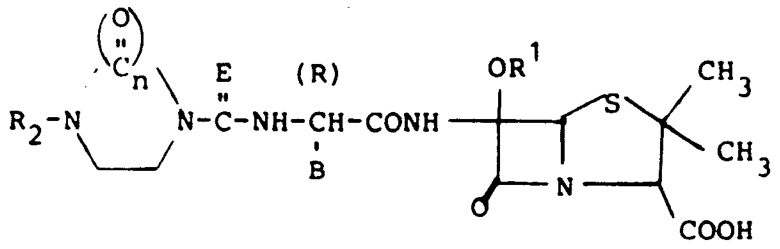

| $R_2$ | n | E | B. | $R_1$ |
|---|---|---|---|---|
| H- | 1 | O | 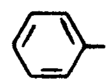 | $-CH_3$ |
| $CH_3$- | 1 | O | " | " |
| $C_2H_5$- | 1 | O | " | " |
| 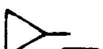 | 1 | O | " | " |
| 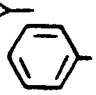 | 1 | O | " | " |
| 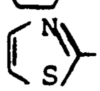 | 1 | O | " | " |
| $(CH_3)_2$CH-S-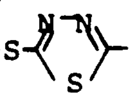 | 1 | O | " | " |
| 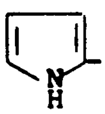-CO- | 1 | O | " | " |
| 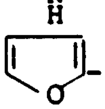-CO- | 1 | O | " | " |
| $CH_3-SO_2$- | 1 | O | " | " |
| 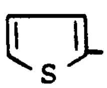-$SO_2$- | 1 | O | " | " |
| 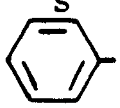-$SO_2$- | 1 | O | " | " |
| H- | 1 | O | HO-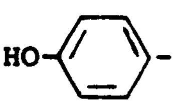- | $-CH_3$ |

| $R_2$ | n | E | B | $R_1$ |
|---|---|---|---|---|
| $CH_3$- | 1 | O | HO-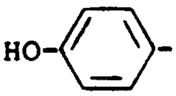 | $-CH_3$ |
| $C_2H_5$- | 1 | O | " | " |
| 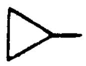 | 1 | O | " | " |
| 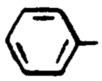 | 1 | O | " | " |
| 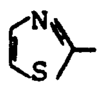 | 1 | O | " | " |
| $(CH_3)_2$-S-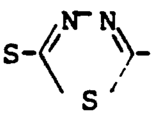 | 1 | O | " | " |
| 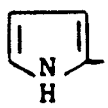 CO- | 1 | O | HO-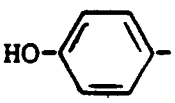 | $-CH_3$ |
| 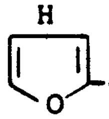CO- | 1 | O | " | " |
| $CH_3-SO_2$- | 1 | O | " | " |
| 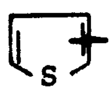$-SO_2$- | 1 | O | " | " |
| 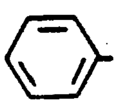$-SO_2$- | 1 | O | " | " |
| H- | 1 | O | 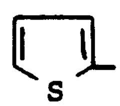 | " |
| $CH_3$- | 1 | O | " | " |
| $C_2H_5$- | 1 | O | " | " |

| $R_2$ | n | E | B | $R_1$ |
|---|---|---|---|---|
| 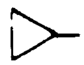 | 1 | O | 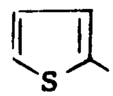 | $-CH_3$ |
| 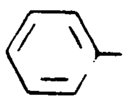 | 1 | O | " | " |
| 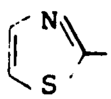 | 1 | O | " | " |
| 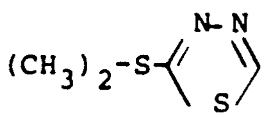 | 1 | O | " | " |
| 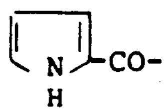 | 1 | O | " | " |
| 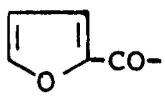 | 1 | O | " | " |
| $CH_3-SO_2-$ | 1 | O | " | " |
| 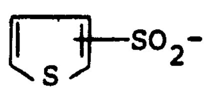 | 1 | O | " | " |
| 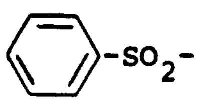 | 1 | O | " | " |
| H- | 1 | O | 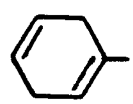 | $-CH_3$ |
| $CH_3-$ | 1 | O | " | " |
| $C_2H_5-$ | 1 | O | " | " |
| 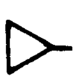 | 1 | O | " | " |

| $R_2$ | n | E | B | $R_1$ |
|---|---|---|---|---|
| 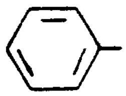 | 1 | O | 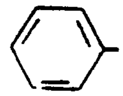 | $-CH_3$ |
| 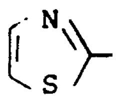 | 1 | O | " | " |
| $(CH_3)_2CH-S-$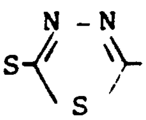 | 1 | O | " | " |
| 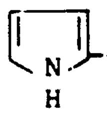-CO- | 1 | O | " | " |
| 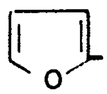-CO- | 1 | O | " | " |
| $CH_3-SO_2-$ | 1 | O | " | " |
| 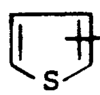$-SO_2-$ | 1 | O | " | " |
| 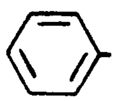$-SO_2-$ | 1 | O | " | " |
| H- | 2 | O | 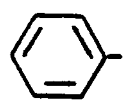 | $-CH_3$ |
| $CH_3-$ | 2 | O | " | " |
| $C_2H_5-$ | 2 | O | " | " |
| 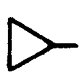 | 2 | O | " | " |
| 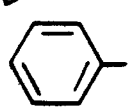 | 2 | O | " | " |

0000380



| $R_2$ | n | E | B | $R_1$ |
|---|---|---|---|---|
| 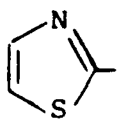 | 2 | O | - | $-CH_3$ |
| $(CH_3)_2CH-S-$ 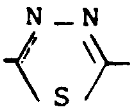 | 2 | O | " | " |
| 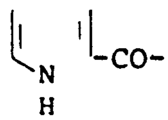 | 2 | O | " | " |
| 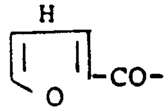 | 2 | O | " | " |
| $CH_3-SO_2-$ | 2 | O | " | " |
| 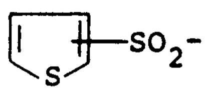 | 2 | O | " | " |
| 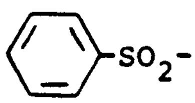 | 2 | O | " | " |
| H- | 2 | O | 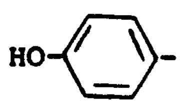 | $-CH_3$ |
| $CH_3-$ | 2 | O | " | " |
| $C_2H_5-$ | 2 | O | " | " |
| 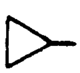 | 2 | O | " | " |
| 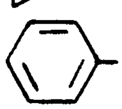 | 2 | O | " | " |

| $R_2$ | n | E | B | $R_1$ |
|---|---|---|---|---|
| 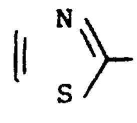 | 2 | O | 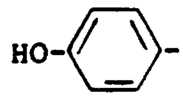 | $-CH_3$ |
| 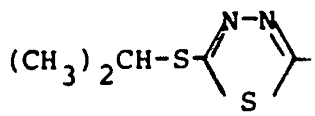 | 2 | O | " | " |
| 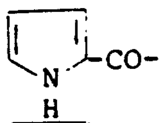 | 2 | O | " | " |
| 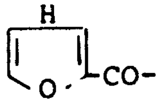 | 2 | O | " | " |
| $CH_3-SO_2-$ | 2 | O | " | " |
| 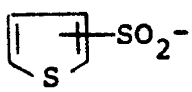 | 2 | O | " | " |
| 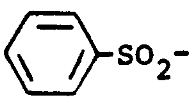 | 2 | O | " | " |
| H | 2 | O | 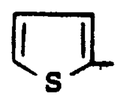 | $-CH_3$ |
| $CH_3-$ | 2 | O | " | " |
| $C_2H_5-$ | 2 | O | " | " |
| 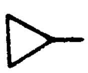 | 2 | O | " | " |
| 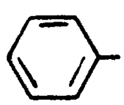 | 2 | O | " | " |
| 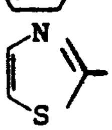 | 2 | O | " | " |

| $R_2$ | n | E | B | $R_1$ |
|---|---|---|---|---|
| $(CH_3CH-S-$ 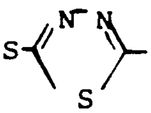 | 2 | O | 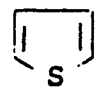 | $-CH_3$ |
| 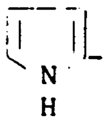 -CO- | 2 | O | " | " |
| 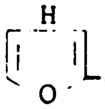 -CO- | 2 | O | " | " |
| $CH_3-SO_2-$ | 2 | O | " | " |
| 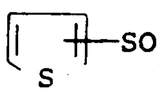 $-SO_2$ | 2 | O | " | " |
| 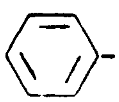 $-SO_2-$ | 2 | O | " | " |
| H- | 2 | O | 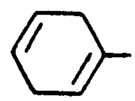 | $-CH_3$ |
| $CH_3-$ | 2 | O | " | " |
| $C_2H_5-$ | 2 | O | " | " |
| 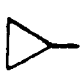 | 2 | O | " | " |
| 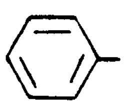 | 2 | O | " | " |
| 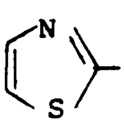 | 2 | O | " | " |

| $R_2$ | n | E | B | $R_1$ |
|---|---|---|---|---|
| $(CH_3)_2CH-S-$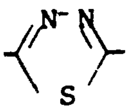 | 2 | O | 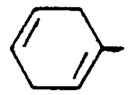 | $-CH_3$ |
| 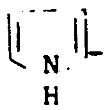$-CO-$ | 2 | O | " | " |
| 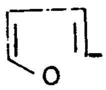$-CO-$ | 2 | O | " | " |
| $CH_3-SO_2-$ | 2 | O | " | " |
| 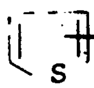$-SO_2-$ | 2 | O | " | " |
| 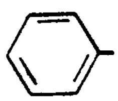$-SO_2-$ | 2 | O | " | " |
| H- | 1 | O | 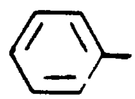 | $-C_2H_5$ |
| $CH_3-$ | 1 | O | " | " |
| $C_2H_5-$ | 1 | O | " | " |
| 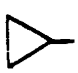 | 1 | O | " | " |
| 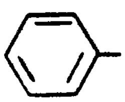 | 1 | O | " | " |
| 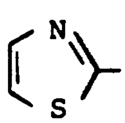 | 1 | O | " | " |
| $(CH_3)_2CH-S-$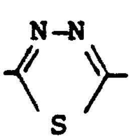 | 1 | O | " | " |

| $R_2$ | n | E | B | $R_1$ |
|---|---|---|---|---|
| 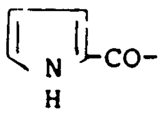 | 1 | O | 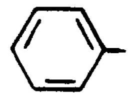 | $-C_2H_5$ |
| 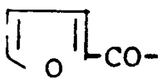 | 1 | O | " | " |
| $CH_3-SO_2-$ | 1 | O | " | " |
| 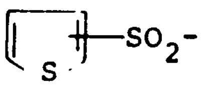 | 1 | O | " | " |
| 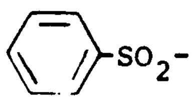 | 1 | O | " | " |
| H- | 1 | O | 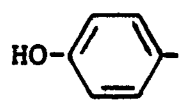 | $-C_2H_5$ |
| $CH_3-$ | 1 | O | " | " |
| $C_2H_5-$ | 1 | O | " | " |
| 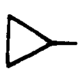 | 1 | O | " | " |
| 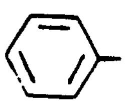 | 1 | O | " | " |
| 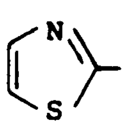 | 1 | O | " | " |
| $(CH_3)_2CH-S-$ 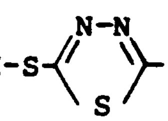 | 1 | O | " | " |

| $R_2$ | n | E | B | $R_1$ |
|---|---|---|---|---|
| 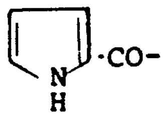 | 1 | O | 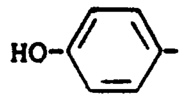 | $-C_2H_5$ |
| 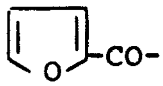 | 1 | O | " | " |
| $CH_3-SO_2-$ | 1 | O | " | " |
| 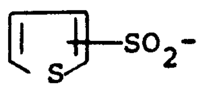 | 1 | O | " | " |
| 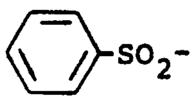 | 1 | O | " | " |
| H- | 1 | O | 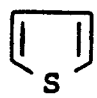 | " |
| $CH_3-$ | 1 | O | " | " |
| $C_2H_5-$ | 1 | O | " | " |
| 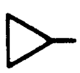 | 1 | O | " | " |
| 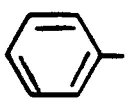 | 1 | O | " | " |
| 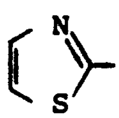 | 1 | O | " | " |
| 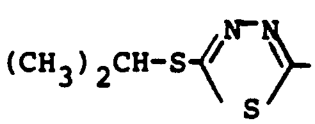 | 1 | O | " | " |
| 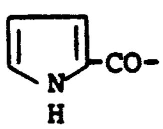 | 1 | O | " | " |

| $R_2$ | n | E | B | $R_1$ |
|---|---|---|---|---|
| 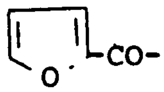 | 1 | O | 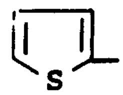 | $-C_2H_5$ |
| $CH_3-SO_2-$ | 1 | O | " | " |
| 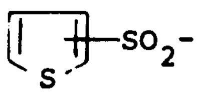 | 1 | O | " | " |
| 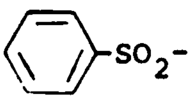 | 1 | O | " | " |
| H- | 1 | O | 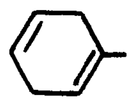 | $-C_2H_5$ |
| $CH_3-$ | 1 | O | " | " |
| $C_2H_5-$ | 1 | O | " | " |
| 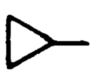 | 1 | O | " | " |
| 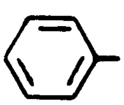 | 1 | O | " | " |
| 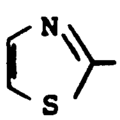 | 1 | O | " | " |
| 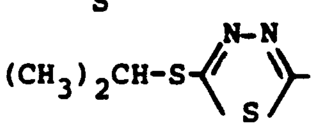 | 1 | O | " | " |
| 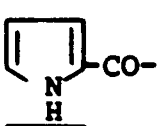 | 1 | O | " | " |
| 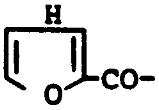 | 1 | O | " | " |

| $R_2$ | n | E | B | $R_1$ |
|---|---|---|---|---|
| $CH_3-SO_2-$ | 1 | O | 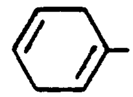 | $-C_2H_5$ |
| 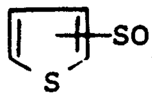$SO_2-$ | 1 | O | " | " |
| 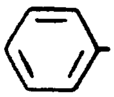$SO_2-$ | 1 | O | " | " |
| H- | 2 | O | 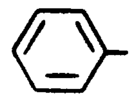 | $-C_2H_5$ |
| $CH_3-$ | 2 | O | " | " |
| $C_2H_5-$ | 2 | O | " | " |
| 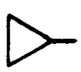 | 2 | O | " | " |
| 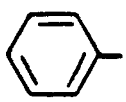 | 2 | O | " | " |
| 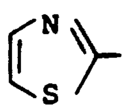 | 2 | O | " | " |
| $(CH_3)_2CH-S-$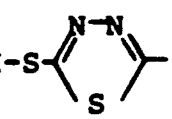 | 2 | O | " | " |
| 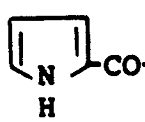CO- | 2 | O | " | " |
| 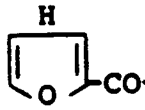CO- | 2 | O | " | " |
| $CH_3-SO_2-$ | 2 | O | " | " |

| $R_2$ | n | E | B | $R_1$ |
|---|---|---|---|---|
| 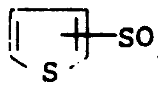 $-SO_2-$ | 2 | O | 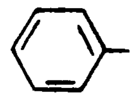 | $-C_2H_5$ |
| 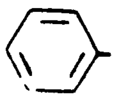 $-SO_2-$ | 2 | O | " | " |
| H- | 2 | O | HO- 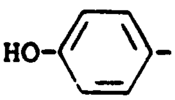 - | $-C_2H_5$ |
| $CH_3-$ | 2 | O | " | " |
| $C_2H_5-$ | 2 | O | " | " |
| 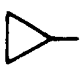 | 2 | O | " | " |
| 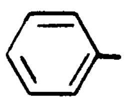 | 2 | O | " | " |
| 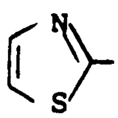 | 2 | O | " | " |
| $(CH_3)_2CH-S-$ 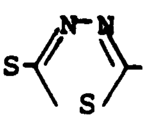 | 2 | O | " | " |
| 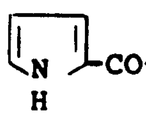 -CO- | 2 | O | " | " |
| 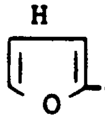 -CO- | 2 | O | " | " |
| $CH_3-SO_2-$ | 2 | O | " | " |

| $R_2$ | n | E | B | $R_1$ |
|---|---|---|---|---|
| 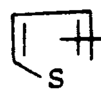$-SO_2-$ | 2 | O | 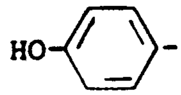 | $-C_2H_5$ |
| 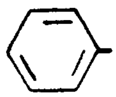$-SO_2-$ | 2 | O | " | " |
| H- | 2 | O | 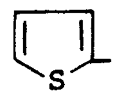 | $-C_2H_5$ |
| $CH_3-$ | 2 | O | " | " |
| $C_2H_5-$ | 2 | O | " | " |
| 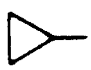 | 2 | O | " | " |
| 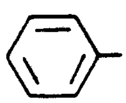 | 2 | O | " | " |
| 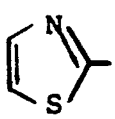 | 2 | O | " | " |
| $(CH_3)_2-S-$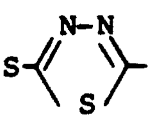 | 2 | O | " | " |
| 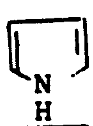-CO- | 2 | O | " | " |
| 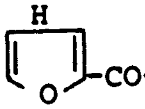-CO- | 2 | O | " | " |
| $CH_3-SO_2-$ | 2 | O | " | " |

| $R_2$ | n | E | B | $R_1$ |
|---|---|---|---|---|
| 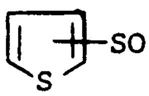$SO_2$- | 2 | O | 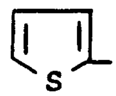 | $-C_2H_5$ |
| 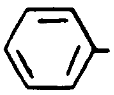$SO_2$- | 2 | O | " | " |
| H- | 2 | O | 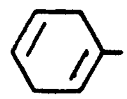 | $-C_2H_5$ |
| $CH_3$- | 2 | O | " | " |
| $C_2H_5$- | 2 | O | " | " |
| 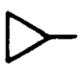 | 2 | O | " | " |
| 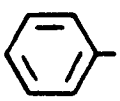 | 2 | O | " | " |
| 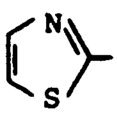 | 2 | O | " | " |
| $(CH_3)_2CH-S-$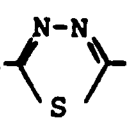 | 2 | O | " | " |
| 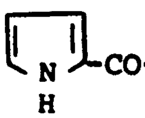CO- | 2 | O | " | " |
| 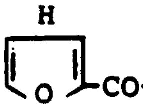CO- | 2 | O | " | " |
| $CH_3-SO_2-$ | 2 | O | " | " |

| $R_2$ | n | E | B | $R_1$ |
|---|---|---|---|---|
| 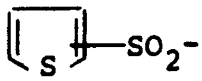 | 2 | O | 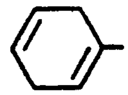 | $-C_2H_5$ |
| 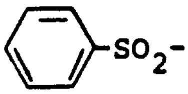 | 2 | O | " | " |
| H | 1 | S | 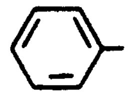 | $CH_3-$ |
| H | 1 | S | 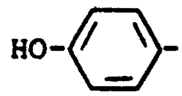 | $CH_3-$ |
| H | 1 | S | 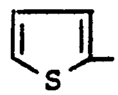 | $CH_3-$ |
| H | 1 | S | 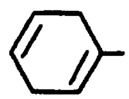 | $CH_3-$ |
| H | 1 | S | 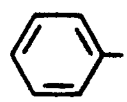 | $C_2H_5-$ |
| H | 1 | S | 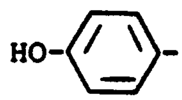 | $C_2H_5-$ |
| H | 1 | S | 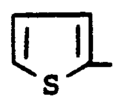 | $C_2H_5-$ |
| H | 1 | S | 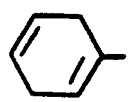 | $C_2H_5-$ |

Die erfindungsgemäßen Wirkstoffe weisen eine starke antimikrobielle Wirksamkeit auf. Diese Eigenschaften ermöglichen ihre Verwendung als Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Wirkstoffe sind gegen ein sehr breites Spektrum von Mikroorganismen wirksam. Mit ihrer Hilfe können z.B. Gram-negative und Gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Wirkstoffe gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Micrococcaceae, wie Staphylokokken, z.B. Staphylococcus aureus, Staph. epidermidis, Staph. aerogenes (Staph. = Staphylococcus);

Lactobacteriaceae, wie Streptokokken, z.B. Streptococcus pyogenes, α- bzw. ß-hämolysierende Streptokokken, nicht (γ -)-hämolysierende Streptokokken, Str. viridans, Str. faecalis (Enterokokken), Str.agalactiae, Str. lactis, Str. equi, Str. anaerobis und Diplococcus pneumoniae (Pneumokokken) (Str. = Streptococcus);

Neisseriaceae, wie Neisserien, z.B. Neisseria gonorrhoeae (Gonokokken), N.meningitidis (Meningokokken), N.catarrhalis und N.flava (N. = Neisseria);

Corynebacteriaceae, wie Corynebakterien, z.B. Corynebacterium diphtheriae, C.pyogenes, C.diphtheroides, C.acnes, C.parvum, C.bovis, C.renale, C.ovis,

Enterobacteriaceae, wie Escherichiae-Bakterien der Coli-Gruppe, Escherichia-Bakterien, z.B. Escherichia coli, Enterobacter-Bakterien, z.B. E.aerogenes, E.cloacae, Klebsiella-Bakterien, z. B. K.pneumoniae, Serratia, z. B. Serratia marcescens (E. = Enterobacter) (K. = Klebsiella), Proteae-Bakterien der Proteus-Gruppe, Proteus, z. B. Proteus vulgaris, Pr. morganii, Pr. rettgeri, Pr. mirabilis (Pr. = Proteus), Providencia z. B. Providencia sp., Salmonelleae, Salmonella-Bakterien, z. B. salmonella paratyphi A und B, S. typhi, S.enteritidis, S.cholerae suis, S.typhimurium (S. = Salmonella), Shigella-Bakterien, z. B. Shigella dysenteriae, (Sh. = Shigella);

Pseudomonadaceae, wie Pseudomonas-Bakterien, z.B. Pseudomonas aeruginosa, Ps.pseudomallei (Ps. = Pseudomonas), Aeromonas-Bakterien, z.B. Aeromonas liquefaciens, A.hydrophila (A. = Aeromonas);
Spirillaceae, wie Vibrio-Bakterien, z.B. Vibrio cholerae, V. proteus, (V. = Vibrio).

Parvobacteriaceae oder Brucellaceae, wie Pasteurella-Bakterien z.B. Pasteurella multocida, Past. pestis (Yersinia), Past. pseudotuberculosis, (Past. = Pasteurella), Haemophilus-Bakterien, z.B. Haemophilus influenzae, (H. = Haemophilus), Bordetella-Bakterien, z.B. B. bronchiseptica (B. = Bordetella).

Pacteroidacea, wie Bacteroides-Bakterien, u.B. Bacteroides fragilis, B. serpens (B. = Bacteroides), Fusiforme-Bakterien, z.B. Fasobacterium fusiforme, Sphaerophorus-Bakterien, z.B. sphaerophorus nocrophorus, Sph. pecroticus, Sph. pyrogenes (Sph. = Sphaerophorus);

Bacillaceae, wie Aerobe Sporenbildner, z.B. Bacillus anthracis, B. subtilis, B. cereus (B. = Bacillus), Anaerobe Sporenbildner-Clostridien, z.B. Clostridium perfrigens, Cl. tetani, Cl. botulinum (Cl. Clostridium);

Die obige Aufzählung von Erregern ist lediglich beispielhaft und keineswegs beschränkend aufzufassen.

Als Krankheiten, die durch die erfindungsgemäßen Wirkstoffe verhindert, gebessert und/oder geheilt werden können, seien beispielsweise genannt:

Erkrankungen der Atmungswege und des Rachenraumes;

Otitis; Pharyngitis; Pneumonie; Peritonitis; Pyelonephritis; Cystitis; Endocarditis; Systeminfektionen; Bronchitis; Arthritis.

Zur vorliegenden Erfindung gehören pharmazeutische Zubereitungen, die neben inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter pharmazeutisch verwendbaren Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z. B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum,Calcium- und Magnesiumstearat und feste Polyäthylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyäthylenglykole, Fette, z.B. Kakaofett und höhere Ester (z.B. $C_{14}$-Alkohol mit $C_{16}$-Fettsäure) und Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyäthylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z.B. Wasser, Äthylalkohol, Isopropylalkohol, Äthylcarbonat, Äthylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Äthylalkohol, Propylenglykol, Suspendiermittel, z.B. äthoxylierte Isostearylalkohole, Polyoxyäthylensorbit- und Sorbitanester, mikrokristalline Cellulose, Aluminiummetahydroxid, Bentonit, Agar-Agar und Tragant oder Gemische oder Stoffe enthalten.

Die genannten Formulierungsformen können auch Färbemittel, Konservierungsstoffe sowie geruchs- und geschmachsverbessernde Zusätze, z.B. Pfefferminzöl und Eukalyptusöl und Süßmittel z.B. Saccharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gewichtsprozent der Gesamtmischung vorhanden sein.

Die oben aufgeführten pharmazeutischen Zubereitungen können ausser den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe nach dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin bei der Verhütung, Besserung und/oder Heilung der oben angeführten Erkrankungen.

Die Wirkstoffe oder die pharmazeutischen Zubereitungen können lokal, oral, parenteral intraperitoneal und/oder rectal, vorzugsweise parenteral, insbesondere intravenös und intramusculös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 6 bis etwa 800, vorzugsweise 15 bis 300 mg/kg Körpergewicht je 24 Stunden, gegebenenfalls in Form mehrerer, z.B. von 3 Einzelgaben zur Erzielung der gewünschten Ergebnisse zu verabreichen. Eine Einzelgabe enthält den oder die erfindungsgemäßen Wirkstoffe, vorzugsweise in Mengen von etwa 2 bis etwa 300, insbesondere 10 bis 150 mg/kg Körpergewicht. Es kann jedoch erforderlich sein, von

den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Im Falle der Anwendung als Futterzusatzmittel können die neuen Verbindungen in üblicher Weise zusammen mit dem Futter bzw. mit Futterzubereitungen oder mit dem Trinkwasser gegeben werden. Dadurch kann eine Infektion durch gram-negative oder gram-positive Bakterien verhindert und ebenso eine bessere Verwertung des Futters sowie die Förderung des Wachstums erreicht werden.
Die Erfindung betrifft demnach auch ein Tierfuttermittel, daß die neuen Penicilline in Anwesenheit von für die Tierernährung verwendbaren Träger- oder Zusatzstoffen enthält sowie ein Verfahren zur Herstellung eines solchen Mittels.

Die neuen Penicilline zeichnen sich durch starke antibakterielle Wirkungen, die in vivo und in vitro geprüft wurden, und durch orale Resorbierbarkeit aus.

Die erfindungsgemäßen Penicilline können zum Zwecke der Erweiterung des Wirkungsspektrums oder zur Wirkungssteigerung z.B. auch mit Aminoglykosidantibiotika wie Gentamicin, Sisomicin, Kanamicin, Amikacin oder Tobramicin kombiniert werden.

Die Wirksamkeit der erfindungsgemäßen Penicilline kann durch die folgenden in vitro- und in vivo-Versuche beispielhaft demonstriert werden:

1. <u>In vitro-Versuche</u>

Die Beispiele zwei, fünf und acht, welche als typische Vertreter der erfindungsgemäßen Verbindungen betrachtet werden können, wurden mit Müller-Hinton-Nährbrühe auf einen Gehalt von 100 µg/ml verdünnt. In der Nährlösung befanden sich jeweils $1 \times 10^5$ bis $2 \times 10^5$ Bakterien pro Milliliter. Die Röhrchen mit diesem Ansatz wurden jeweils 24 Stunden bebrütet und anschließend wurde der Trübungsgrad bestimmt. Trübungsfreiheit gibt Wirkung an. Bei der Dosierung von 100 µg/ml waren die folgenden Bakterienkulturen trübungsfrei (sp.= species) :

Klebsiella pneumoniae; Enterobacter aerogenes sp.; Providencia; Serratia marcescens; E.coli BE; Salmonella sp.; Shigella sp.; Proteus, indolnegativ und indolpositiv; Pasteurella pseudotuberculosis; Haemophilus influenzae; Bordetella bronchiseptica; Staphylococcus aureus 133; Neisseria catarrhalis sp.; Diplococcus pneumoniae sp.; Streptococcus pyogenes W.; Enterococcus sp.; Lactobacillus sp.; Corynebacterium diphteriae gravis; Corynebacterium pyogenes M; Clostridium tetani; Pseudomonas aeruginosa sp.

2. In vivo-Versuche

Aus der folgenden Tabelle 1 geht die Wirkung einer der erfindungsgemäßen Verbindungen gegen eine Reihe von Bakterien im Tierversuch mit der weißen Maus hervor. Die weißen Mäuse vom Stamm $CF_1$ wurden intraperitoneal mit der jeweils angegebenen Bakterienart infiziert.

Tabelle 1

Tierversuche mit der weißen Maus

Bestimmung der $ED_{100}$ nach 24 Stunden

| Keim | Dosis in mg des Penicillins der Beispiele 1, 2, 9, pro kg/Körpergewicht (subcutan) |
|---|---|
| Escherichia coli C 165 | 2 x 150 |
| Klebsiella 63 | 2 x 150 |

**Therapie : zweimalig : 30 und 90 Minuten nach der Infektion. Die $ED_{100}$ ist die Dosis, bei der 100% der infizierten Tiere nach 24 Stunden noch überleben.**

Beispiel 1

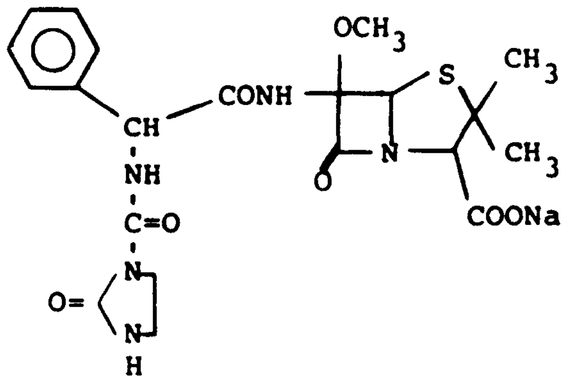

Unter Stickstoff legt man 150 Vol. Teile THF und 0,57 Gew.-Teile Lithiumhydrid gelöst in 100 Vol. Teilen Methanol bei -70°C vor und gibt 8,3 Gew.-Teile D-α-(Imidazolidin-2-on-1-yl-carbonylamino)-benzylpenicillin hinzu. Direkt anschließend fügt man schließlich noch 5,9 Gew.-Teile t-Butylhypochlorit zu. Dann rührt man 4 h bei -60°C weiter.

Die Aufarbeitung erfolgt durch Eingabe der Reaktionslösung in 200 Vol. Teile Wasser unter gleichzeitiger Zugabe von verdünnter Salzsäure, so daß der pH Wert bei ca. 7,5 bleibt.

Nach Extraktion organischer Verunreinigungen mit Essigester, Sauerstellen (pH 1,8) und Extraktion des Produktes mit Essigester unterschichtet man letztere Essigester-Phasen mit Wasser und gibt langsam unter Kühlung soviel verdünnte Natronlauge hinzu, bis man einen pH-Wert von 6,5-7,5 erhält.

Diese wäßrige Phase wird gefriergetrocknet. Man erhält 7,2 (78 %) 6-α-Methoxy-6-ß-[D-2-(imidazolidin-2-on-1-yl-carbonylamino)-phenylacetamido]-penicillinsäure (Natriumsalz).

Das Dünnschichtchromatogramm zeigt eine praktisch saubere Verbindung. (Als Laufmittelsystem wurde verwendet: 200 ml n-Butylacetat/36 ml n-Butanol/100 ml Essigsäure, geschüttelt mit 60 ml Phosphatpuffer pH 7, $\frac{1}{15}$ M. Verwendet wurde die organische Phase).
Das IR-Spektrum enthält die notwendige ß-Lactambande bei 1760 $cm^{-1}$. 100 MHz $^{1}H$ - NMR - Spektrum (Aceton/$D_2O$) (Shift-Werte $\delta$, TMS als innerer Standard:

0,8 s 3H, 1,1 s 3 H, 3,1-3,3 m 2H, 3,3 s 3 H, 3,5-3,65 m 2 H (etwas verdeckt durch $H_2O$-Signal). 3,85 s 1 H, 5,3 s 1 H, 5,4 s 1H, 7,2 m 5 H.

Beispiel 2

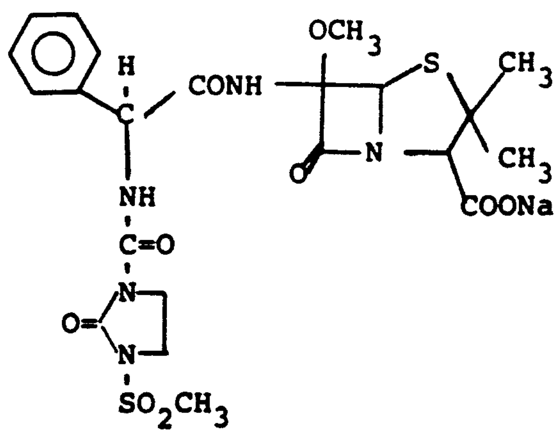

Arbeitsweise analog Beispiel 1.

Eingesetzt wurden:

22,5 Gew.-Teile D-α-[3-(Methylsulfonyl)-imidazolidin-2-on-1-yl-carbonylamino]-benzylpenicillin

1,27 Gew.-Teile Lithiumhydrid
4,34 Gew.-Teile t-Butylhypochlorit
500 Vol. Teile TMF und
500 Vol. Teile Methanol.

Reaktionszeit 2 h bei -60°C.

Ausbeute 20,1 Gew.-Teile (85 %) 6-α-Methoxy-6-ß-/D̄-2-(3-methylsulfonyl)-imidazolidin-2-on-1-yl-carbonylamino̲7-phenylacetamido-penicillansäure (Natriumsalz)

DC: einheitliche Verbindung, IR-Spektrum: ß-Lactam-Bande bei 1760 $cm^{-1}$

NHR-Spektrum ($D_2O$)

1,2 s 3 H, 1,6 s 3 H, 3,6 s 3 H, 3,8 s 3 H, 4,1 m 4 H, 4,4 s 1 H, 5,7 s 1 H, 5,8 s 1 H, 7,2 breites s 5 H.

Beispiel 3

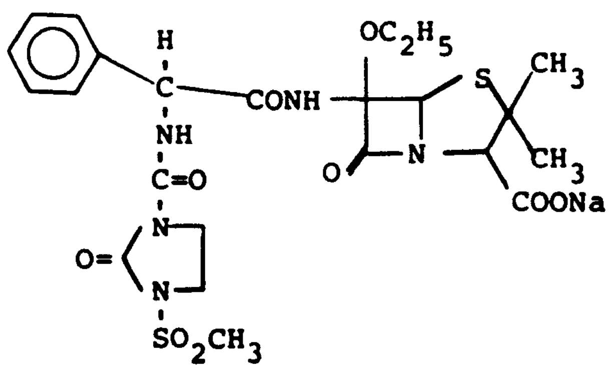

Arbeitsweise analog Beispiel 1.

Ansatz:

8,1 Gew.-Teile D-α-/3-(Methylsulfonyl)-imidazolidin-2-on-1-yl-carbonylamino-benzylpenicillin
0,48 Gew.-Teile Lithiumhydrid
32 Gew.-Teile t-Butylhypochlorit
80 Vol. Teile THF
100 Vol. Teile Methanol

Reaktionszeit 2 h bei -60°C
Ausbeute: 7,5 Gew.-Teile (82 %) 6-α-Äthoxy-6-ß-/D-2-ß-(3-methylsulfonyl)-imidazolidin-2-on-1-yl-carbonylamino/-phenylacetamido/-penicillansäure (Natriumsalz)
DC: enthält noch etwa Ausgangsmaterial, sonst saubere Verbindung (ca. 90 %ig) mit deutlich größerem $R_f$-Wert als die Ausgangsverbindung.
IR: 1760 $cm^{-1}$
NMR: 1,0 s 3 H, 1,2 s 3 H, 1,3 s doppeltes t 3 H, 3,3 s 3 H, darunter verdeckt m 2 H, 3,8 m 4 H, 4,05 s 1 H, 5,45 s 1 H, 5,5 s 1 H, 7,4 m 5 H.

Beispiel 4

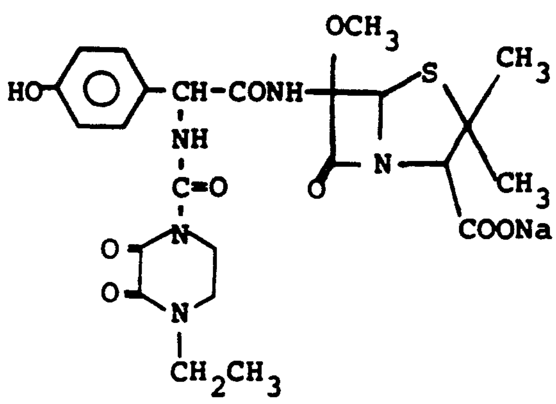

Versuchsdurchführung siehe Beispiel 1.

Ansatz:

3 Gew.-Teile 6[D-α-(4-Alkyl-2,3-dioxo-1-piperazino-carbonyl-amino)p-hydroxyphenyl-acetamido]-penicillansäure
1,3 Vol. Teile t-Butylhypochlorit
120 Vol. Teile TMF
120 Vol. Teile $CH_3OH$
0,22 Gew.-Teile LiH

Reaktionszeit 3 h bei $-60^o$ bis $-70^oC$

Ausbeute 2,3 Gew.-Teile (75 %) 6-α-Methoxy-6-ß-[D-2-(4-äthyl-2,3-dioxopiperazinocarbonylamino)p-hydroxyphenyl-acetamido] penicillansäure (Natriumsalz)

IR-Spektrum: 1750 $cm^{-2}$
(Nujol)

NMR-Spektrum: 1,0-1,6 m 3H+3H+3H, 3,3-3,9 m 4H+2H+3H,
(d-Aceton/$D_2O$) 4,1 s 1H (etwas verdeckt durch Lösungsmittel), 5,6 breites s 1H+1H, 7,o-7,6 m 4H.

<u>Beispiel 5</u>

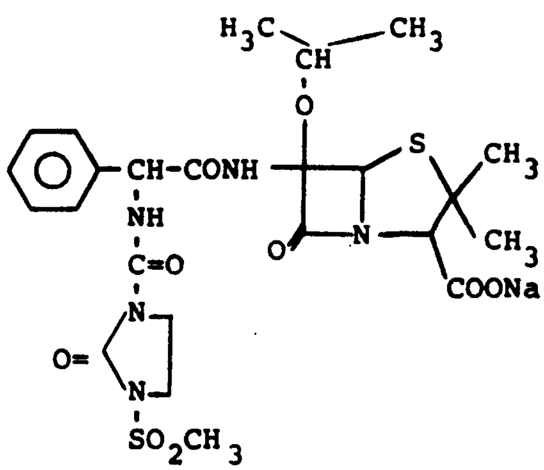

Versuchsdurchführung wie Beispiel 1.

Ansatz:

10,8 Gew.-Teile D-α-[3-(Methylsulfonyl)imidazolin-2-on-1-yl carbonylamino]-benzylpenicillin
100 Vol. Teile THF
48,75 Vol. Teile einer 15 %igen Lösung von n-Butyllithium in n-Hexan
100 Vol. Teile i-Propanol
2,17 Gew.-Teile t-Butylhypochlorit

Die Alkoholatlösung wird zweckmäßig vorher getrennt aus Butyllithium und i-Propanol hergestellt und dann zur gekühltem THF-Lösung gegeben.
Reaktionszeit 4,5 h bei -60°C
Ausbeute: 8,8 Gew. (71 %) 6-α-Isopropoxy-6-ß-[D-2-[3-(methylsulfonyl)imidazolidin-2-on-1-yl-carbonylamino]phenylacetamido]-penicillansäure (Natriumsalz)
IR-Spektrum: Schulter bei 1760 $cm^{-1}$ (Nujol)
NMR-Spektrum: 0,95 s 3H, 1,1-1,25 doppeltes Duplett 3H, 1,33 s 3H, 1,55 d 3H, 3 = 4Hz, 3,35 s 3H, darunter verdeckt Septett 1H, 3,9 breites s 4H, 4,00 s 1H (letztere beiden Signale teilweise durch Lösungsmittel überdeckt) 5,49 s 1H, 5,5 s 1H, 7,2-7,6 m 5H

Beispiel 6

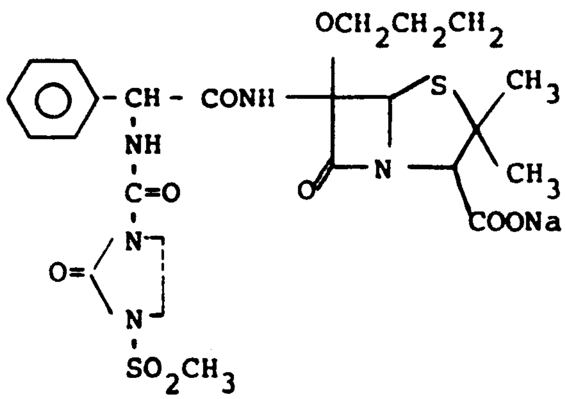

Arbeitsweise wie in Beispiel 1.

Ansatz:

| | | |
|---|---|---|
| 8,1 | Gew.-Teile | D-α-[3-(Methylsulfonyl)-imidazolidin-2-on-1-yl-carbonylaminobenzylpenicillin |
| 36 | Vol. Teile | einer 15 %igen Lösung von n-Butyllithium in n-Hexan |
| 100 | Vol. Teile | THF |
| 100 | Vol. Teile | n-Propan |
| 3,2 | Gew.-Teile | t-Butylhypochlorit |

Vorher wird wieder die Alkoholatlösung hergestellt (siehe Beispiel 5)

Reaktionszeit: 2 h bei -60° bis -70°C

Ausbeute: 5,5 Gew.-Teile (60 %) 6-α-(1-Propoxy)-6-β-[D-2-[3-(methylsulfonyl)imidazolidin-2-on-1-yl-carbonylamino]-phenylacetamido]-penicillansäure (Natriumsalz)

Beispiel 7

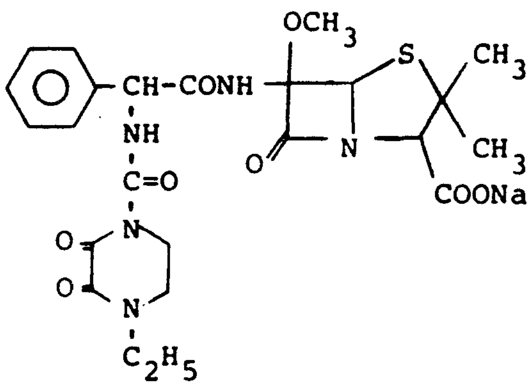

Gearbeitet wurde wie in Beispiel 1 beschrieben.

Ansatz:

2,2 Gew.-Teile 6/D-α-(4-Äthyl-2,3-dioxo-1-piperazino-carbonylamino)phenylacetamidopenicillansäure
0,48 Vol.Teile t-Butylhypochlorit
100 Vol. Teile THF
100 Vol. Teile $CH_3OH$
0,13 Gew.-Teile Lithiumhydrid

Reaktionszeit 3 h bei -70°C

Die Ausfällung als Natriumsalz erfolgte, indem man die eingeengte Essigesterlösung nach der sauren Extraktion) in eine ätherische/methanolische Lösung von Natrium, 2-Äthylhexanoat, die 2/3 der theoretischen Menge an Natrium-2-äthylhexanoat enthielt, unter kräftigem Rühren gab.

Der Niederschlag wurde abgesaugt und nach 20 Minuten in 10 % $CH_3OH$/Äther nachgerührt. Nach nochmaligem Filtrieren erhielt man 1,3 Gew.-Teile 6-α-Methoxy-6-ß-[D-2-(4-Äthyl-2,3-dioxo-1-piperazinocarbonylamino]-phenylacetamido] penicillansäure (Natriumsalz).

Beispiel 8

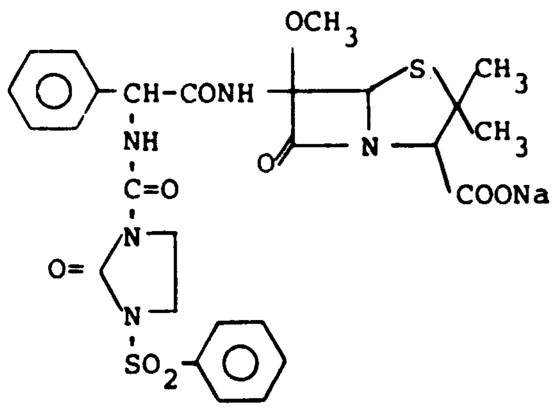

Arbeitsweise siehe Beispiel 1.

Ansatz:

9 Gew.-Teile D-α-[3-(Phenylsulfonyl)imidazolidin-2-on-1-yl-carbonylamino]-benzylpenicillin.
0,48 Gew.-Teile Lithiumhydrid
1,6 Gew.-Teile t-Butylhypochlorit
100 Vol. Teile $CH_3OH$
100 Vol. Teile THF

Ausbeute: 7,2 Gew.-Teile (73,5 %) 6-α-Methoxy-6-ß-[D-2-[3-(phenylsulfonyl)imidozilidin-2-on-1-yl-carbonylamino]-phenylacetamido]-penicillansäure (Natriumsalz)

DC: einheitlich, IR-Spektrum (Nujol), ß-Lactambande bei 1760 $cm^{-1}$
NHR Spektrum
(d-Aceton/$D_2O$) 1,0 s 3H, 1,3 s 2H, 3,4 s 3H, 3,9 m 4H, 4,0 s 1H, 5,4 s 1H, 5,5 s 1H, 7,2-5,0 m 10H.

Beispiel 9

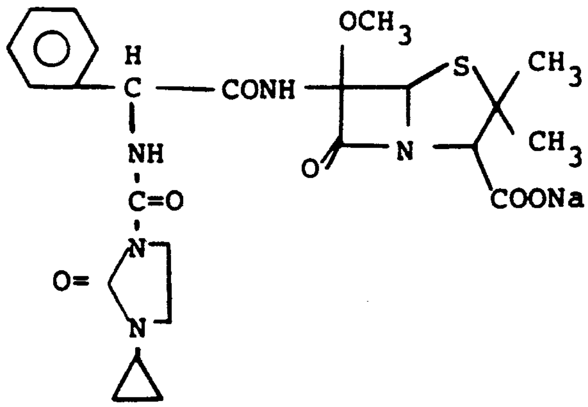

Arbeitsweise wie Beispiel 1.

Ansatz:

2,5 Gew.-Teile D-α-[3-(Cyclopropyl)-imidazolidin-2-on-1-yl-carbonylamino]benzylpenicillin
0,16 Gew.-Teile LiH
0,54 Gew.-Teile Butylhypochlorit
50 Vol. Teile THF
50 Vol. Teile $CH_3OH$

Reaktionszeit 3 h bei -60° bis -70°C
Ausbeute: 1,9 Gew.-Teile (68 %) 6-α-6-ß-/D-2-(3-Cyclopropyl-imidazolidin-2-on-1-yl-carbonylamino)phenylacetamido/-penicillansäure (Natriumsalz)
DC: einheitlich, IR (Nujol): 1760 $cm^{-1}$
NHR-Spektrum ($CD_3OD$): 0,75 d 4H, 0,9 s 3H, 1,3 s 3H, 2,45 p 1H, 3,3 m 2H, 3,45 s 3H, 3,65 m 2H, 4,05 s 1H, 5,4 s 1H, 5,5 s 1H, ca. 7,3 m 5H.

Beispiel 10

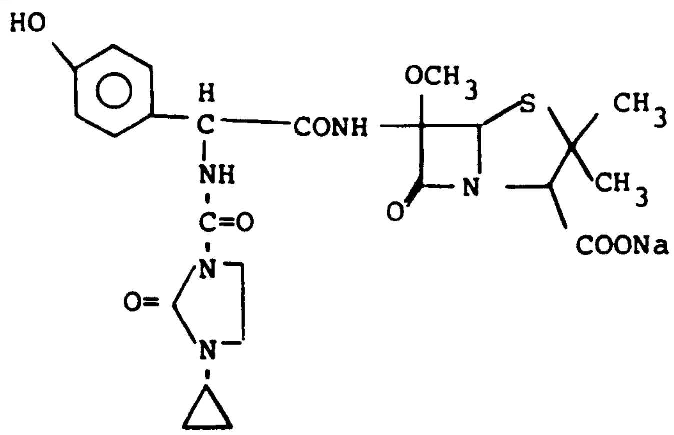

Versuchsdurchführung siehe Beispiel 1.

Ansatz:

1,92 Gew.-Teile 6/D-α-(3-Cyclopropyl-imidazolidin-2-on-1-yl-carbonylamino)p-hydroxyphenyl-acetamido/ penicillansäure)
0,15 Gew.-Teile Lithiumhydrid
0,8 Gew.-Teile t-Butylhypochlorit
50 Vol. Teile THF
50 Vol. Teile $CH_3OH$

Reaktionszeit 3,5 h bei -65°C Ausbeute: 1,5 Gew.-Teile (71 %) 6-α-Methoxy-6-β-/D-2-(3-cyclopropyl-imidazolidin-2-on-1-yl-carbonylamino)p-hydroxyphenyl-acetamido/-penicillansäure (Natriumsalz) DC: einheitlich; IR (Nujol) : 1760 $cm^{-1}$ NMR ($CD_3OD$): 0,73 d 4H, 1,0 s 3H, 1,35 s 3H, 3,5 p 1H, 3,35 m 2H, 3,45 s 3H, 3,7 m 2H, 4,1 s 1H, 5,4 s 1H, 5,5 s 1H, 6,8 m 2H, 7,35 m 2H.

Beispiel 11

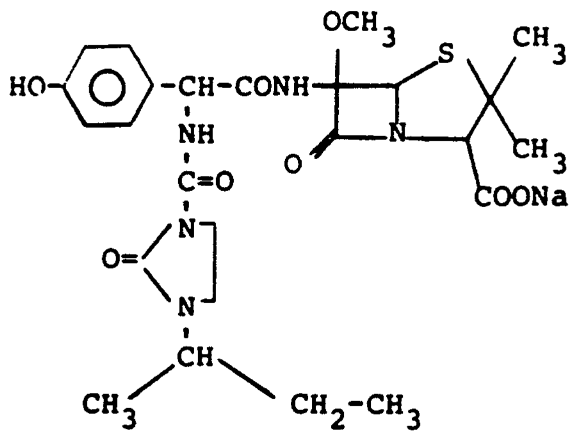

Ausbeute 68 % 6-α-Methoxy-6-β-{D-2-/3-(1-methyl-1-propyl-)imidazolidin-2-on-1-yl-carbonylamino/-p-hydroxyphenylacetamido} penicillansäure (Natriumsalz) DC: einheitlich, IR (Nujol): 1760 $cm^{-1}$ NMR-Spektrum ($CD_3OD$): 0,85 t 3H, 1,1 m 6H, 1,5 m 5H, 3,4 m 2H, 3,5 s 3H, 3,7 m 2H, 4,1 s 1H, 5,4 s 1H, 5,5 s 1H, 6,8 m 2H, 7,3 m 2H.

Beispiel 12

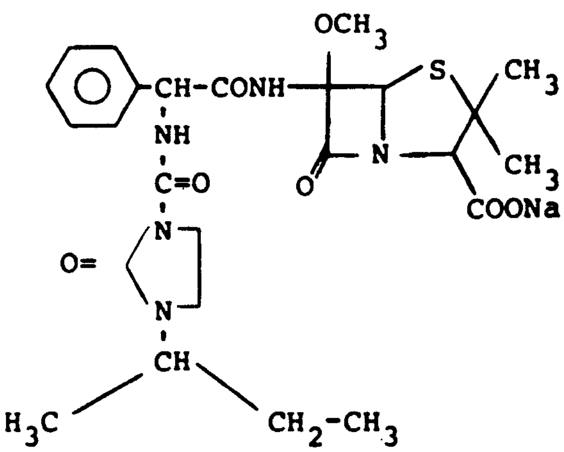

Ausbeute: 64 % 6-α-Methoxy-6-β-{D-2-[3-(1-Methyl-1-propyl)-imidazolidin-2-on-1-yl-carbonylamino]-phenylacetamido}-penicillansäure (Natriumsalz)
IR-Spektrum (Nujol): Bande bei 1785 $cm^{-1}$
Das NMR-Spektrum entspricht bis auf die veränderten aromatischen Protonensignale dem aus Beispiel 11.

Beispiel 13

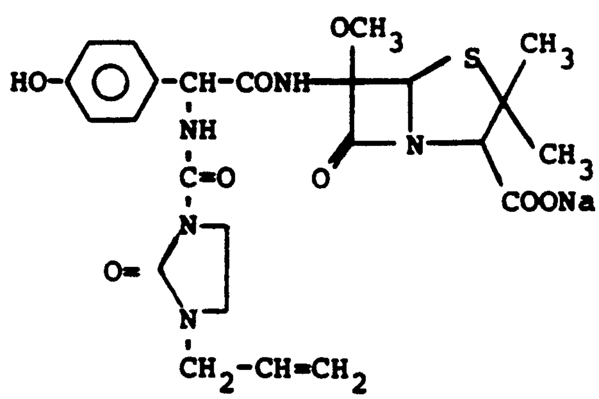

Ausbeute 62 % 6-α -Methoxy-6-ß-{ D-2-[3-(1-Prop-2-enyl) imidazolidin-2-on-1-yl-carbonylamino]-p-hydroxyphenyl-acetamido } -penicillansäure (Natriumsalz)
DC: fast einheitlich ca. 90 %ig; IR-Spektrum: 1762 $cm^{-1}$
Das NMR-Spektrum stimmt mit der angenommenen Struktur überein.

Beispiel 14

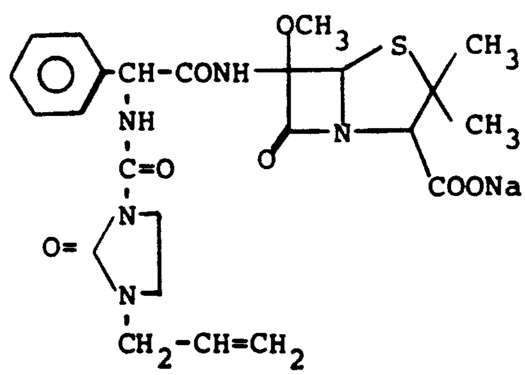

Ausbeute 72 % 6 ß{D-2-[3-(1-Prop-2-enyl)imidazolidin-2-on-1-yl-carbonylamino]phenylacetamido } -6-α-methoxy-penicillansäure (Natriumsalz)
DC: einheitliche Verbindung; IR-Spektrum (Nujol): 1765 $cm^{-1}$
NMR-Spektrum ($CD_3OD$): 0,9 s 3H, 1,15 s 3H, 3,4 m 2H (teilweise verdeckt durch Lösungsmittel) 3,5 s 3H, 3,9 m 2H+2H, 4,05 s 1H, 5,0-5,6 m 3H, 5,4 s 1H, 5,5 s 1H, 7,4 m 5H.

Beispiel 15

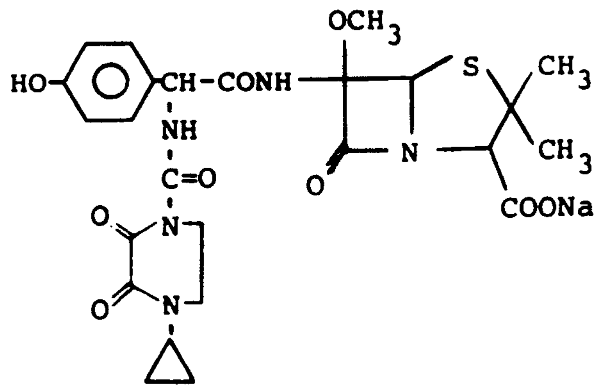

Arbeitsweise wie in Beispiel 1 beschrieben.

Ansatz:

2,-5 Gew.-Teile 6[D-α-(4-Cyclopropyl-2-oxo-1-piperazino-carbonylamino)p-hydroxyphenyl-acetamido]penicillansäure
0,18 Gew.-Teile Lithiumhydrid
0,98 Gew.-Teile t-Butylhypochlorit
50 ml absolutes THF
50 ml absolutes $CH_3OH$
Reaktionszeit 3 h bei ca. -65°C.
DC: einheitlich; IR (Nujol): starke Bande bei 1760 $cm^{-1}$
NMR ($CD_3OD$): 0,75 d 4H, 1,05 s 3H, 1,4 s 3H, 2,8 p 1H, 3,3 m 4H, 3,45 s 3H, 4,05 s 1H, 5,4 s 1H, 5,5 s 1H, 6,75 m 2H, 7,3 m 2H.
Ausbeute: 1,5 Gew.-Teile (56 %) 6-α-Methoxy-6-β-[D-2-(4-Cyclopropyl-2,3-dioxo-1-piperazinocarbonylamino)p-hydroxyphenylacetamido]-penicillansäure (Natriumsalz)

Beispiel 16

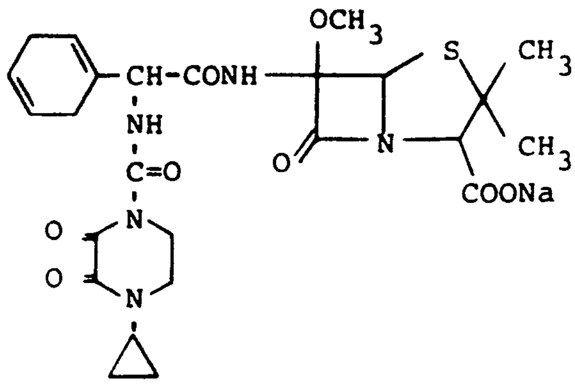

Ausbeute: 58 % 6-α-Methoxy-6β[D-2-(4-Cyclopropyl-2,3-dioxo-1-piperazinocarbonylamino)1,4-cyclohexadienylacetamido] penicillansäure (Natriumsalz)

DC: ca. 90 % enthält, 2 kleine Verunreinigungen, IR-Spektrum: 1760 $cm^{-1}$

NMR ($CD_3OD$): 0,7 d 4H, 0,9 s 3H, 1,15 s 3H, 3,65 breites 4H, 3,8 (teilweise verdeckt) m 1H, 3,2 m 2H, 3,4 s 3H, 3,65 m 2H, 4,05 s 1H, 5,5 s 1H, 5,6 s 1H, 5,6-5,8 (teilweise verdeckt) m 3H.

Beispiel 17

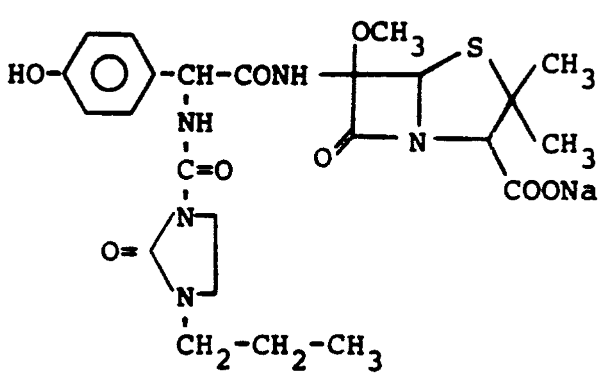

Ausbeute: 64 % 6-α-Methoxy-6-β-{D-2-[3-(1-propyl)-imidazolidin-2-on-1-yl-carbonylamino]p-hydroxyphenyl-acetamido} penicillansäure (Natriumsalz)
DC: einheitlich, IR-Spektrum: 1765 $cm^{-1}$.

Beispiel 18

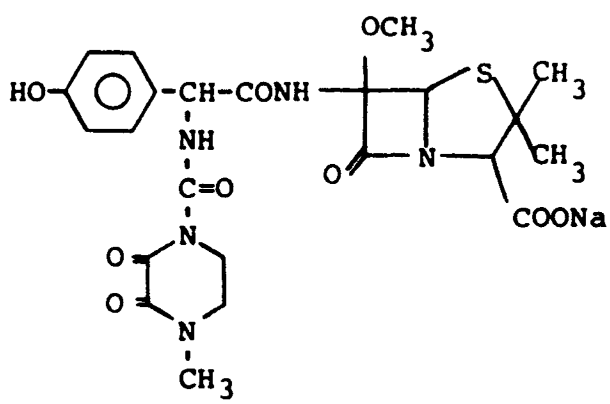

Ausbeute: 64 % 6-α-Methoxy-6-β-[D-2-(3-methylimidazolidin-2-on-1-yl-carbonylamino)p-hydroxyphenylacetamido]penicillansäure (Natriumsalz)
DC: praktisch einheitlich; IR-Spektrum: Bande bei 1765 $cm^{-1}$.

Patentansprüche

1. Verbindungen der Formel (I)

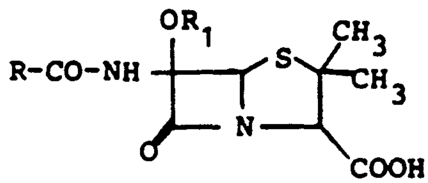

(I)

in der

$R_1$ Wasserstoff, gegebenenfalls substituiertes Alkyl, gegebenenfalls substituiertes Alkenyl, Alkinyl mit der Dreifachbindung in der 2-Stellung, gegebenenfalls substituiertes Cycloalkyl, gegebenenfalls substituiertes Aralkyl, gegebenenfalls substituiertes Aryl, gegebenenfalls substituiertes Heterocyclylalkyl oder gegebenenfalls substituiertes Heterocyclyl bezeichnet;

R für einen Rest der Formeln (II) oder (IIa) steht

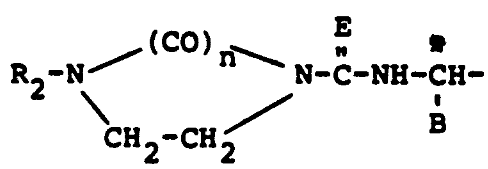

(II)

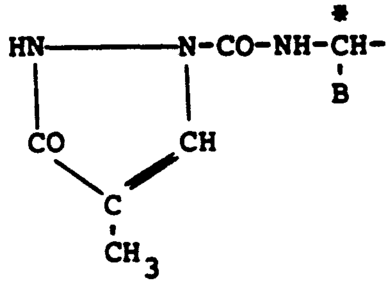

(IIa)

in denen

$\overset{*}{C}$ ein Chiralitätszentrum symbolisiert, das in der R- oder S-Form vorliegen kann,

E Sauerstoff oder Schwefel bezeichnet,

B für gegebenenfalls substituiertes Phenyl, 1-Cyclohexenyl-(1) oder 1,4-Cyclohexadienyl-(1) steht,

n 1 oder 2 bezeichnet und

$R_2$ für den Fall, daß n = 1 und $R_1$ = Methyl ist, gegebenenfalls substituiertes Alkyl, gegebenenfalls substituiertes Alkenyl, mit der Doppelbindung in der 2-, 3-, 4- oder 5-Stellung, Alkinyl mit der Dreifachbindung in der 2-, 3- oder 4-Stellung oder einen Rest der Formeln (III) oder (IV) bezeichnet

$R_3$-CO- (III) $R_4$-$SO_2$- (IV)

in denen

$R_3$ Wasserstoff oder Niederalkoxy bezeichnet,

$R_3$,$R_4$ gegebenenfalls substituiertes Alkyl, gegebenenfalls substituiertes Alkenyl mit der Doppelbindung in der 2-, 3-, 4- oder 5-Stellung, Alkinyl mit der Dreifachbindung in der 2-, 3- oder 4-Stellung, Amino,

Niederalkylamino, Diniederalkylamino, in dem die beiden Alkylreste direkt oder über ein Heteroatom unter Bildung eines heterocyclischen Ringes verbunden sein können, gegebenenfalls substituiertes Cycloalkyl, gegebenenfalls substituiertes Aralkyl, gegebenenfalls substituiertes Aryl, einen gegebenenfalls substituierten 3- bis 6-gliedrigen heterocyclischen Ring, der O, S und/oder N als Heteroatome enthalten kann, bedeuten und

$R_2$ für den Fall, daß n = 2 und $R_1$ von Methyl verschieden ist für Wasserstoff, gegebenenfalls substituiertes Alkyl, gegebenenfalls substituiertes Alkenyl, Alkinyl mit der Dreifachbindung in der 2-Stellung, gegebenenfalls substituiertes Cycloalkyl, gegebenenfalls substituiertes Aralkyl, gegebenenfalls substituiertes Aryl, gegebenenfalls substituiertes Heterocyclylalkyl, gegebenenfalls substituiertes Heterocyclyl, wobei der heterocyclische Ring in beiden Fällen 3-, 4-, 5- oder 6-gliedrig sein und O, S und/oder N als Heteroatome enthalten kann, bezeichnet sowie die Salze, insbesondere die pharmazeutisch verwendbaren Salze, der Verbindungen gemäß Formel (I).

2. Verbindung der Formel

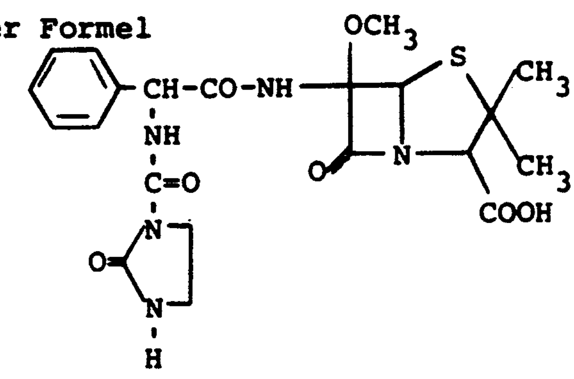

und ihre pharmazeutisch verwendbaren Salze.

3. Verbindung der Formel

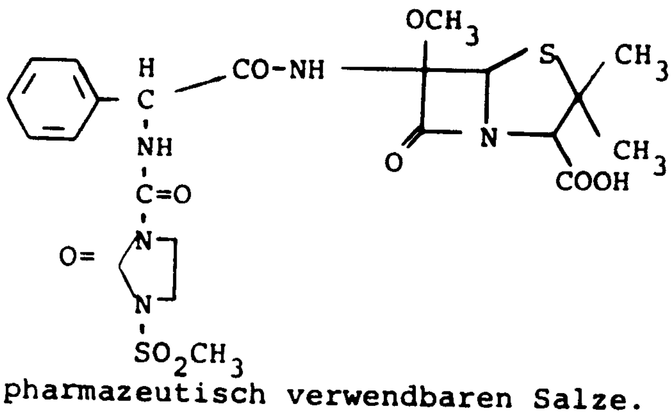

und ihre pharmazeutisch verwendbaren Salze.

4. Natriumsalze der Verbindungen gemäß den Ansprüchen 1 bis 19.

5. Verfahren zur Herstellung von Verbindungen der Formel (I)

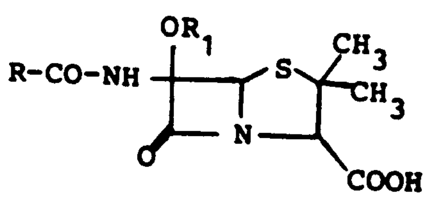

(I)

in der

R und $R_1$ die oben angegebene Bedeutung besitzen, sowie deren Salzen, dadurch gekennzeichnet, daß man Verbindungen der Formel (V)

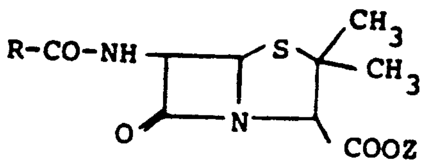

(V)

in der

R die oben angegebene Bedeutung besitzt und

Z Wasserstoff, Acetoxymethyl, eine leicht entfernbare esterbildende Gruppe oder den kationischen Rest einer Base bezeichnet, mit 2-10 Äquivalenten pro Äquivalent des Penicillins einer Base in Anwesenheit eines Überschusses eines Alkohols der Formel $R_1OH$, in der $R_1$ die oben angegebene Bedeutung besitzt, in einem inerten organischen Lösungsmittel umsetzt, zwischen 1 bis 8 Äquivalente eines N-Halogenierungsmittels zufügt und die Verbindung der Formel (I) gegebenenfalls nach vorheriger Abspaltung der Säureschutzgruppe und/oder Überführung in ein Salz, isoliert.

6. Pharmazeutische Zubereitung, gekennzeichnet durch einen Gehalt an einer Verbindung gemäß den Ansprüchen 1 bis 20.

7. Verfahren zur Herstellung einer pharmazeutischen Zubereitung gemäß Anspruch 22, dadurch gekennzeichnet, daß man Verbindungen gemäß den Ansprüchen 1 bis 20 mit inerten, pharmazeutisch geeigneten Träger- und/oder Zusatzstoffen vermischt.

8 . Tierfuttermittel gekennzeichnet durch einen Gehalt an einer Verbindung gemäß den Ansprüchen 1 bis 20.

9 . Verfahren zur Herstellung eines Tierfuttermittels gemäß Anspruch 24, dadurch gekennzeichnet, daß man eine Verbindung gemäß Anspruch 1 bis 20 mit für die Tierernährung verwendbaren Träger- und/oder Zusatzstoffen vermischt.

10. Verwendung von Verbindungen gemäß den Ansprüchen 1 bis 20 bei der Bekämpfung von bakteriellen Erkrankungen bei Mensch und Tier.

11. Verwendung von Verbindungen gemäß den Ansprüchen 1 bis 20 zur Förderung des Wachstums und zur Verbesserung der Futterauswertung bei Tieren.

12. Verfahren zur Behandlung von bakteriellen Erkrankungen, dadurch gekennzeichnet, daß man Verbindungen gemäß Ansprüchen 1 bis 20 Menschen oder Tieren appliziert, die diese Krankheiten haben.

Europäisches Patentamt

# EUROPÄISCHER RECHERCHENBERICHT

Nummer der Anmeldung

0000380

EP 78 10 0322

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE - A - 2 528 078 (BAYER)<br>* Patentansprüche 1, 14-16 * | 1,6,7,<br>10,12 |
| | DE - A - 2 320 039 (BAYER)<br>* Patentansprüche 1,3,48,49 * | 1,6,7,<br>10,12 |
| A | DE - A - 2 456 307 (BAYER)<br>* Patentansprüche 1,27-30 * | 1,6-12 |

KLASSIFIKATION DER ANMELDUNG (Int.Cl.²)

C 07 D 499/02
A 61 K 31/43
A 23 K 1/17

RECHERCHIERTE SACHGEBIETE (Int. Cl.²)

C 07 D 499/02
C 07 D 499/00
A 61 K 31/43
A 23 K 1/17

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 29-09-1978 | CHOULY |